# EUROPEAN PATENT SPECIFICATION

(11) **EP 1 501 572 B1**
(45) Date of publication and mention of the grant of the patent: **08.08.2007**
(21) Application number: 03716610.5
(22) Date of filing: 17.03.2003
(51) Int. Cl.: A61M 5/142, A61M 5/172

(54) **PATIENT CONTROLLED ACTIVATION WITH IMPLANTABLE DRUG DELIVERY DEVICES**
PATIENTENKONTROLLIERTE AKTIVIERUNG VON IMPLANTIERBAREN VORRICHTUNGEN ZUR VERABREICHUNG VON ARZNEIEN
DISPOSITIFS IMPLANTABLES D'ADMINISTRATION DE MEDICAMENTS COMMANDES PAR LE PATIENT

(30) Priority: 26.04.2002 US 133665
(43) Date of publication of application: 02.02.2005
(73) Proprietor: MEDTRONIC, INC., Minneapolis, MN 55432 (US)
(72) Inventor: Ullestad, David, C., Maple Grove, MN 55311 (US); Ali, Irfan Zafer, Woodbury, MN 55125 (US); Kovach, Peter J., Fridley, MN 55432 (US); Bourget, Duane, Albertville, MN 55301 (US)
(74) Representative: Hughes, Andrea Michelle
(86) International application number: PCT/US2003/008017
(87) International publication number: WO 2003/090821

(56) References cited:
- WO-A-01/34220
- DE-A- 3 227 518
- US-A- 4 731 051
- US-A1- 2001 037 083

## Description

### FIELD OF THE INVENTION

The present invention relates to a patient controlled, implantable drug infusion system for administering medicine. More particularly, the present invention provides a system for administering analgesia to a patient that allows the patient to control the analgesia infusion rates within limits prescribed by a physician for that particular patient. The present invention further provides a closed loop patient feedback system that links information pertaining to a patient's physical assessment and activity level with a particular drug therapy.

### BACKGROUND OF THE INVENTION

Patient-controlled analgesia systems have become an effective and popular means of providing analgesia to patients with postoperative pain. A typical patient-controlled analgesia system contains an external pump with a microcomputer programmed to give small amounts of pain medication, for instance, 1 mg of morphine intravenously at pre-set intervals or at a low continuous rate. In addition, typical systems may be programmed to give small amounts of pain medication every time a patient pushes a button on the end of a cable. As a result of patient-controlled analgesia systems, patients require less pain medication and experience improved post-operative pain relief.

Current analgesia systems, however, have limitations that constrain a patient's ability to tailor the analgesic dose or drug therapy according the patient's own requirements. For instance, conventional systems provide drug therapies that are dependent on a time-of-day or a known schedule of need or do not allow time-dependent programs to be temporarily altered based on patient need. In addition, these systems do not allow the patient to receive information from the system pertaining to the system's current operating status. Moreover, these systems often require frequent trips to the physician for checkups and infusion rate adjustments.

U.S. Patent No. 4,731,051, Fischell, Programmable Control Means For Providing Safe and Controlled Medication Infusion, discloses an implantable programmable infusion pump (IPIP) according to the preamble of claim 1, including a fluid reservoir filled with selected medication, a pump for causing a precise volumetric dosage of medication to be withdrawn from the reservoir and delivered to the appropriate site within the body and a control means for actuating the pump in a safe and programmable manner. The control means includes a microprocessor, a permanent memory containing a series of fixed software instructions, and a memory for storing prescription schedules, dosage limits and other data. The microprocessor actuates the pump in accordance with programmable prescription parameters and dosage limits stored in the memory. A communication link allows the control means to be remotely programmed. The control means incorporates a running integral dosage limit and other safety features which prevent an inadvertent or intentional medication overdose. The control means also monitors the pump and fluid handling system and provides an alert if any improper or potentially unsafe operation is detected.

Furthermore, conventional systems do not allow the patient to internally record information pertaining to the patient's self-assessment (i.e., physical state and activity level). As a result, these systems fail to provide a method of linking a patient's feedback information to the infusion/therapy being delivered at the time the patient entered the self-assessment information into the system. Instead, to link these drug therapies to the patient's physical state or activity level, the physician generally waits until a regularly scheduled appointment to ask the patient questions regarding the patient's personal assessment on how they felt or activity level during the drug therapy program. Thus, physicians must rely solely on the patient's memory when making decisions to adjust the patient's therapy or prescription-an unreliable and costly method of evaluating the effectiveness of the drug therapy.

Accordingly, there is a need for a drug infusion system and method that addresses the above shortcomings. The present invention solves this need by providing an improved patient controlled analgesia system, a system that also provides a closed loop feedback to directly link information pertaining to a patient's physical assessment and activity level with a particular drug therapy.

### BRIEF SUMMARY OF EXEMPLARY EMBODIMENTS

The present invention provides a programmable system for allowing patients to control their own therapy or drug infusion within limits set by a physician. The system comprises a pump mechanism for dispensing medicine to a patient in a format controlled by a control system within the pump mechanism. The control system is programmed to provide a patient control over pump functions selected or prescribed by the physician, thereby allowing the patient to have input into his or her daily dosage regimen. Patient control over pump functions leads to more accurate drug therapy and fast control over drug overdose or underdose.
Initially, the pump is presented to the physician in a default state where all pump functions are disabled. The physician configures the pump by enabling the desired pump functions and inputting the parameters for each function. Physician programming of the pump is done via programmer downlink or telemetry. Once programmed into the pump, the functions and related parameters are managed by the pump's control system. Depending on the physician's configuration of the pump, certain pump functions may be available to a patient via a hand held control device that communicates with the pump via telemetry. For example, the pump functions that a physician may make available to the patient via the patient control device may include: stop pump; re-start pump; increase current pump rate; decrease current pump rate; patient bolus, stop/cancel patient bolus, status summary; test pump alarm; silence pump alarm; and set patient event. The physician may program the parameters for each of the above listed functions to keep the therapy or drug infusion within physician prescribed parameters. The patient may then administer a bolus infusion and/or increase or decrease their infusion rate to vary their therapy based on their current/planned activity or personal assessment of "how they are feeling."

In addition to providing patient control within physician prescribed limits, the system has a patient feedback mechanism that allows a patient to record information into the pump control system pertaining to the patient's self-assessment. For instance, the patient can use the hand held control device to enter information corresponding to the patient's physical state or activity level at the time of the drug infusion (e.g. "I feel terrible right now", "I feel good right now", "I am active at this time", "I am resting at this time"). The information is time/date stamped according to the pump mechanism's current time/date and recorded in the pump mechanism's control system. In turn, the patient feedback information can be downloaded and reviewed by the physician at the next clinical visit. The physician can use the patient feedback mechanism to link the patient's feedback information to the infusion/therapy being delivered at the time the patient entered the information into the pump. The patient feedback mechanism provides valuable patient information to the physician that is used at a later date without requiring the patient to remember or note the activity.

Furthermore, the pump mechanism may be programmed to maintain the status of all, or a selected subset of patient controlled pump functions and the patient feedback mechanism so that the status can be readily accessible to the physician. The physician may upload and analyze the data to adjust patient drug refill appointments based on patient activity - resulting in less frequent appointments and less drug waste. In turn, convenience to the patient is increased since less travel is needed for appointments and drug adjustments. As a result, drug therapies are enhanced, and patient and physician convenience is increased.

### BRIEF DESCRIPTION OF THE DRAWINGS

Figure 1 is an exemplary embodiment of the external programming device and the external patient control communicating with the pump mechanism via telemetry;
Figure 2 depicts exemplary functions of the pump mechanism;
Figure 3 is graph illustrating the operation of the pump mechanism over a 24 hour period;
Figure 4 is an exemplary embodiment of the external patient control device;
Figure 5 is flow chart of an exemplary operation of the present invention;
Figure 6 depicts a method for linking information pertaining to a patient's physical assessment and activity level to a particular drug therapy routine.

### DESCRIPTION OF EXEMPLARY EMBODIMENTS

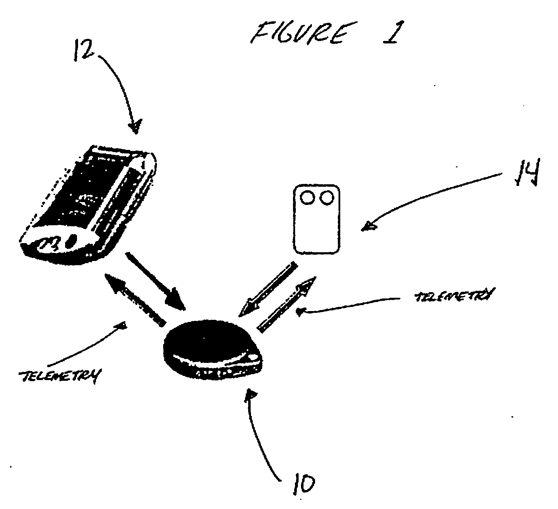

Referring to Figure 1, an exemplary embodiment of the present invention provides a system for delivering analgesia to a patient in accordance with preset parameters prescribed or programmed by a physician. Specifically, the present invention employs an implantable, programmable pump mechanism 10 having a control system that controls the operation of the pump mechanism. The control system includes a microprocessor and a memory programmable with selected functions for controlling the operation of the pump mechanism. The memory stores the programs and data related to the operation of the pump mechanism. The memory is coupled to the microprocessor, which in turn runs the desired operating programs that control the operation of the pump mechanism.

Access to the microprocessor is provided though a communications port located in the pump mechanism. The communication port receives and transmits information from/to an external programming device 12 or an external patient control device 14 via telemetry. Figure 1 depicts the pump mechanism 10 and the external programming device 12 and patient control device 14 communicating via telemetry. This feature allows for the downloading and uploading of any or all information from the memory of the microprocessor to the programming devices.

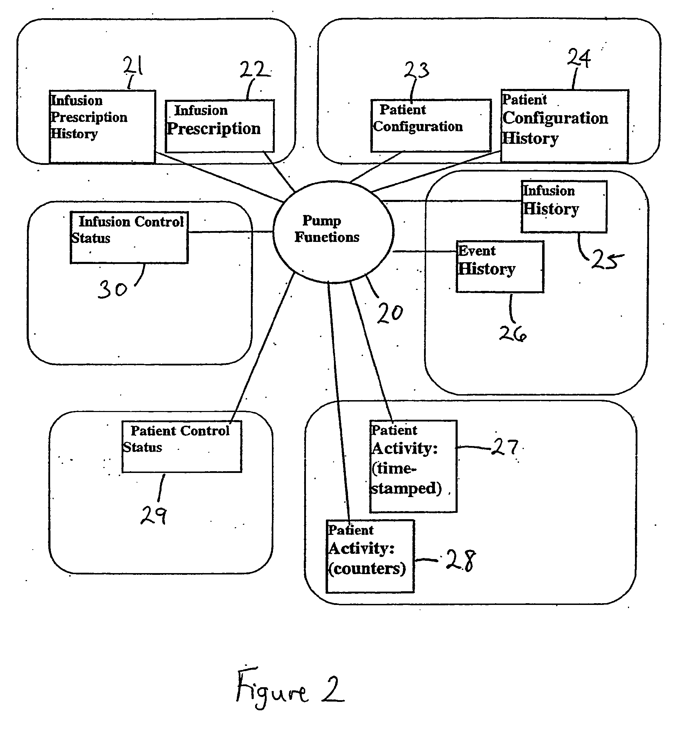

Referring to Figure 2, exemplary functions of the control system of the pump mechanism 10 are generally depicted. The function of the control system 20 include generally infusion prescription history 21, infusion prescription 22, patient configuration 23, patient configuration history 24, infusion history 25, event history 26, time-stamped patient activity 27, patient activity counter 28, patient control status 29, and infusion control status 30. The infusion prescription history 21, infusion prescription 22, patient configuration 23, and patient configuration history 24 are entered via the programmer 12 and downlinked into the pump mechanism. The infusion history 25, event history 26, patient activity time-stamped 27, and patient activity counter 28 are functions of the pump mechanism that are managed by the pump and are accessible via the programmer 12. These functions are able to be cleared by the programmer 12. The patient control status 29, and infusion control status 30 are also functions of the pump mechanism that are managed by the pump and are accessible via the programmer 12. However, these functions are not alterable by the programmer 12. The control system functions 20 of the pump mechanism are described in greater detail below. Initially, the system of the present invention is presented to a physician in an initial default state where all pump functions are disabled. Using the external programming device 12, the physician programs the initial patient infusion prescription. The prescription may be a fixed rate prescription that does not vary over time, unless changed by the physician or patient. Alternatively, the prescription may be multi-step where the infusion rate automatically adjusts over time. The infusion prescription history (a record of a given number of past prescriptions) is stored in the memory of the pump mechanism and may be managed and retrieved via the external programming device 12.

The physician also programs the pump mechanism to have a specific patient configuration by selecting which pump functions are enabled and selecting the parameters for each enabled pump function. If the physician enables a pump function, the physician must specify all of the programmable parameters associated with that pump function since the pump assumes no default values. The patient configuration containing the pump functions and related parameters is stored in the memory of the pump mechanism and can only be changed by the physician. The patient configuration can be read, cleared, or adjusted by the physician using the external programming device 12.

When the patient configuration is programmed into the pump mechanism, the patient configuration is time stamped according to the pump mechanism's current time/date setting contained in the pump mechanism's memory. As a result, the physician may easily identify when the patient configuration was entered or programmed into the pump mechanism. Should the physician need to enter a new patient configuration into the pump mechanism, the previous or old patient configuration is copied and recorded in a patient configuration history stored in the pump mechanism's memory. Each time the physician downlinks or programs a new patient configuration into the pump mechanism, the old or previous patient configuration, including all time/date stamped information, is automatically stored in the patient configuration history.

Depending on the patient configuration prescribed by the physician, certain pump functions may be available to the patient via the external patient control device 14. Like the external programming device 12, the external patient control device 14 is a hand held controller that communicates with the pump mechanism via telemetry. The pump functions that the physician may make available to the patient via the patient control device 14 may include: stop pump; re-start pump; increase current pump rate; decrease current pump rate; patient bolus, stop/cancel patient bolus, status summary; test pump alarm; silence pump alarm; and set event. These pump functions are explained in greater detail below. Some or all of these functions are available to the patient and may be used by the patient to vary their therapy based on their current/planned activity or personal assessment of "how they are feeling."

The physician may set the "count period" associated with all the patient controlled commands. As described below, the count period is a programmable period of time (commonly a 24-hour day or a 90-day refill cycle) set by the physician in which the number of times an internally set patient event has occurred can be compared against a maximum number that are allowed to occur. All command counts are reset at the beginning of the count period. The physician sets a maximum number of "counts" for each patient command. If the patient tries to exceed the maximum count number, the pump mechanism will lockout and prevent the patient from further using the particular command until the end of the count period. In an alternative embodiment, the count period may be a fixed count period window where the patient may perform a set number of actions within a specific time period, such as a 24-hour period, and the counts reset at the end of the time period. In another alternative embodiment, the count period may be a sliding window count period where a set number of actions are counted within a specific time period, such as a 24-hour period, and any actions occurring more than 24-hours ago are no longer counted.

The stop pump function allows the patient to stop the pump (pump rate = 0) and cancel any bolus infusions. The physician may program a specific time period or maximum duration that the pump will remain stopped, for example one hour. After the specified time period lapses, the pump resumes its function. Typically, the maximum time period that the pump will be allowed to remain off will not exceed the time in which pump damage may occur due to inactivity for certain peristaltic pumps. The "tube set" period is the maximum time period that the pump will be allowed to remain off.

Alternatively, the physician may allow the patient to stop the pump for an unlimited time period to preserve pump battery power. In other words, the pump resumes function only upon receiving a re-start pump command by the patient or physician. Table 1 contains configuration parameters associated with the patient stop pump function. In an exemplary embodiment, the numbers chosen for parameter limits are based on a computer's digital mathematics and data storage formats. For example, a single-byte (8-bit) integer number ranges from 0-255 (2⁸ - 1) and a 2-byte (16 bit) integer number ranges from 0 - 65,535 (2¹⁶ - 1).

| **TABLE 1** | | |
|---|---|---|
| **Parameters** | **Description** | **Range** |
| Enable/Disable | Function is available for patient's use if "Enabled". | 0 - Disabled |
| | | 1 - Enabled |
| Max Duration | The maximum amount of time the pump is allowed to be kept off. Typically, this will be set at the pump's "Tube Set" period, or no limit. | 0 - 65,534 Minutes (a timed duration), and "Forever" value 65,535 (no limit, not timed) |
| Lockout Duration | The minimum amount of time between restart and stop | 0 - 65,534 Minutes (a timed duration), and "Forever" value 65, 535 (no limit, not timed) |
| Stop Count | The maximum number of stops per "Count Period" | 0 - 65,534, and a "No Limit" value (65,535) |

The patient re-start pump function cancels the stop pump command issued by the patient and restarts the pump. The patient re-start pump command, however, cannot restart a pump that has been stopped by a physician. After receiving a re-start pump command, the pump resumes pumping at the rate determined by the infusion prescription, the timing of which continued in the background while the pump was stopped.

The increase current rate and decrease current rate functions allow the patient to switch between different pump rates provided the pump is programmed with a fixed rate prescription. Depending on the physician programmed patient configuration, the patient may switch between a number of different pump rates. In an exemplary embodiment of the invention, the pump mechanism has at least ten (10) different rates that may be specified to be above or below the starting infusion rate in any combination. The physician may program the pump to allow all of the ten different rates to be used or a subset of the ten different rates to be used. For example, the pump mechanism may be programmed to allow rate adjustments 5 above and 5 below the starting rate; 1 above and 2 below the starting rate; 3 above and 0 below the starting rate; etc. When a patient sends an increase current rate or decrease current rate command to the pump, the pump mechanism changes its pump rate and will pump at the new rate until the patient instructs the pump mechanism otherwise. Thus, once the patient switches to a different rate, the patient does not have to periodically instruct the pump mechanism to continue pumping at the new rate. In other words, the pump mechanism never reverts back to any previous pumping rate if not periodically updated by the patient. When the increase rate and decrease rate function is enabled for a patient, the infusion prescription is no longer used. If the physician does not enable this function, the pump will pump at the prescription set by the physician and the patient will not be allowed to increase or decrease the rate.

The physician may limit the patient's ability to switch between different rates by programming the pump mechanism to have a lockout duration or interval and a maximum rate deviation. The lockout duration or lockout interval limits the number of times a rate change may be made within a given period of time specified by the physician. In other words, the lockout duration or lockout interval is the interval of time after the last rate change during which a subsequent change rate request is denied by the pump mechanism. Essentially, this function allows the physician to control the rate at which the patient is permitted to modify the prescription rate.

In addition, the patient may also be prohibited from making a rate change based on the combination of the count period and the maximum rate deviation parameters. The maximum rate deviation is the maximum number of rates that the patient can deviate from the reference rate (the rate that the pump mechanism is pumping at the start of the count period) within one count period. The maximum rate deviation function limits the patient such that the patient may only modify their rate by a certain amount within a count period. For example, a physician may program the pump to have a 6 hour count period and to allow the patient to deviate from the reference rate by a maximum of 2 rates. If the patient requests a rate that is three rates above or below the reference rate, the pump mechanism will reject the request and not make the requested rate change.

The lockout duration and the maximum rate deviation functions are further exemplified in the following example. In this example, the physician programmed the patient configuration as follows:

| **TABLE 2** | | |
|---|---|---|
| | Count Period | 1440 minutes (24 hours) |
| | Lockout Duration | 180 minutes (3 hours) |
| | Max Rate Deviation | 2 |
| | Start Rate Index | 4 |
| 0 | INC/DEC Rate 0 | Unused |
| 1 | INC/DEC Rate 1 | Unused |
| 2 | INC/DEC Rate 2 | Unused |
| 3 | INC/DEC Rate 3 | 50 mcl/day |
| 4 | INC/DEC Rate 4 | 100 mcl/day (start rate index) |
| 5 | INC/DEC Rate 5 | 200 mcl/day |
| 6 | INC/DEC Rate 6 | 400 mcl/day |
| 7 | INC/DEC Rate 7 | 800 mcl/day |
| 8 | INC/DEC Rate 8 | 1 ml/day |
| 9 | INC/DEC Rate 9 | 2 ml/day |
| 10 | INC/DEC Rate 10 | 4 ml/day |

As shown in Table 2, the physician programmed the pump to have a 24-hour count period that, for example, starts at 6 a.m. each day. The physician programmed the pump to have a maximum rate deviation of 2 within any count period. The physician selected which increase/decrease (INC/DEC) rates are available to the patient and the pump rates that correspond to each INC/DEC rate. In this example, the physician selected rates that allow the patient to increment 6 rates above the start rate, and 1 rate below start rate. The physician left INC/DEC rates 0-2 unused and unavailable to the patient. In addition, the physician chose INC/DEC rate 4 as the start rate index (the start rate index is simply the pump rate in which the physician selects as the starting pump rate, which in this case is 100 mcl/day).

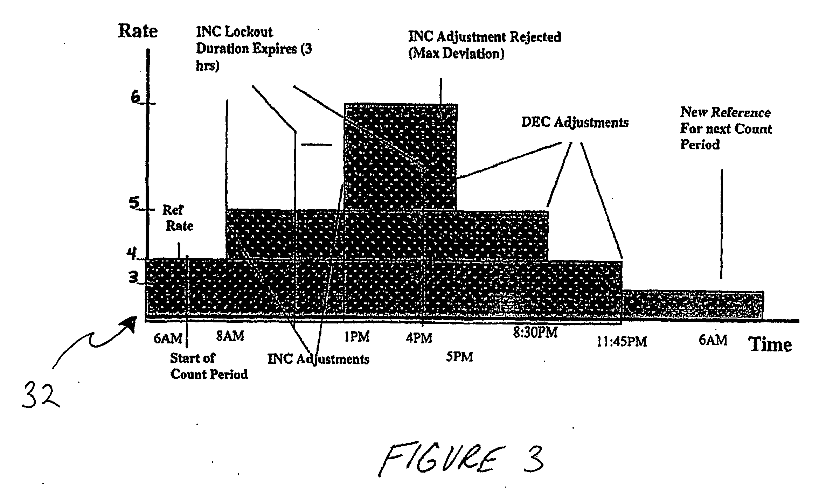

Referring to Figure 3, there is shown an exemplary INC/DEC rate diagram 32. As depicted, the reference rate (the pump rate at the start of the count period) for Day 1 is INC/DEC rate 4 at 100 mcl/day and the count period starts at 6 a.m. At 8 a.m. the patient decides that they would like to increase the current rate from INC/DEC rate 4 to INC/DEC rate 5. The pump accepts the request and increases the current rate to INC/DEC rate 5. Any additional increase rate command between 8 a.m. and 11 a.m. will be rejected until the 3 hour lockout duration expires. At 1 p.m., the patient requests a rate increase to INC/DEC rate 6. The pump accepts the patient's request and increases the current rate to INC/DEC rate 6. Any increase rate command between 1 p.m. and 4 p.m. will be rejected until the 3 hour lockout duration expires. At 4:30 p.m. the patient attempts to increase the rate from INC/DEC rate 6 to rate 7. Even though the lockout period expired at 4 p.m., the request is rejected since the patient already incremented 2 rates above the reference rate (INC/DEC rate 4) during the 24 hour count period. This increase was rejected by the maximum rate deviation function. It should be understood that the patient is permitted to decrease immediately the pump rate following any rate increase. Likewise, the patient is also permitted to increase immediately the pump rate following any rate decrease.

At 5 p.m. the patient successfully decreases the current rate from INC/DEC rate 6 to INC/DEC rate 5. At 8:30 p.m. the patient successfully decreases the current rate from INC/DEC rate 5 to INC/DEC rate 4. Similarly, at 11:45 p.m. the patient successfully decreases the current rate from INC/DEC rate 4 to rate 3. This example demonstrates that the maximum rate deviation function limits the patient to 2 rates above or below the reference rate for the count period, not just 2 increments or decrements. In other words, the patient could have decremented the rate all the way down to INC/DEC rate 2, since INC/DEC rate 4 is the reference rate for this count period.

A new 24 hour count period begins on Day 2 at 6 a.m. A new reference rate is set at the beginning of each count period. The new reference rate is set according to the current rate at the beginning of the count period. For example, since the patient did not make any rate changes after 11:45 p.m. on Day 1, the current rate remained at INC/DEC rate 3 until the start of the count period starting on Day 2. Thus, the reference rate for the count period starting on Day 2 is INC/DEC rate 3 (50 mcl/day).

In addition to the increase current pump rate function and decrease current pump rate function, the physician may program the pump to allow the patient to command an immediate injection of a high quantity of analgesia, i.e., a patient bolus. The patient bolus is used to increase the level of analgesia when there is inadequate analgesia and before the patient can benefit from new settings to address the higher analgesic requirement. The physician sets the patient bolus to be administered at a preset rate (bolus rate) and a preset duration (bolus period). When the bolus period is completed, the infusion rate returns to the rate that was in effect prior to commanding the patient bolus. The patient will be prohibited from commanding a patient bolus while an existing patient bolus is in progress. Similarly, the patient will be prohibited from commanding a rate increase or decrease during a bolus period. The patient, however, may be permitted to command an increased or decreased rate after a patient bolus is completed.

The physician may limit the patient's ability to command a patient bolus by programming the pump mechanism to have a timed bolus lockout and a bolus count lockout. Specifically, the physician may program the patient configuration to have a bolus lockout whereby the pump will only accept a specific number of patient bolus requests within a specified time period, for example a 3 hour time period. In addition, the physician may program the patient configuration to have a bolus count lockout whereby the pump mechanism will only accept a specific number of patient bolus requests within the count period. The lockout timers associated with the INC/DEC lockout duration function and the maximum rate deviation function are not affected by a patient bolus.
Table 3 contains configuration parameters associated with the patient bolus.

| **TABLE 3** | | |
|---|---|---|
| **Parameters** | **Description** | **Range** |
| Enable/Disable | Function is available for patient's use if Enabled | 0 - Disabled |
| | | 1 - Enabled |
| Bolus Rate | Rate to be delivered when activated | Any valid rates supported by the pump |
| | activated | by the pump |
| Bolus Duration | Period of time the bolus rate is in effect | 1 - 65,534 minutes (a timed duration) or "forever" value of 65,535 which means the bolus is not timed and therefore has no limit. |
| Bolus Lockout Duration | The minimum amount of time between accepted bolus commands | 1 - 65,535 minutes |
| Bolus Count | The maximum number of boluses per count period | 1 - 65,534 or a no limit value of 65,535 |

Similarly, the physician may program the pump to have a stop bolus function that allows the patient to cancel a patient bolus and return to the pump rate prior to the patient bolus command. The stop bolus command, however, does not reset the timed bolus lockout or the bolus count lockout functions. In other words, once a patient commands a patient bolus that activates the timed bolus lockout or the bolus count lockout functions, the lockouts will stay in effect even though the patient may choose to cancel the patient bolus.

The system of the present invention may also be equipped with audible alarms corresponding to each pump function that alert the patient that a pump function or programmed parameter has been exceeded. In turn, the physician may program the pump to allow the patient to test the pump's audible alarm and to silence or unsilence the pump's audible alarm. The test pump alarm function simply allows the patient to test the audible alarm to ensure that the alarm in functioning properly. Table 4 contains configuration parameters associated with the test pump alarm function.

| **TABLE 4** | | |
|---|---|---|
| **Parameters** | **Description** | **Range** |
| Enable/Disable | Function is available for patient's use if enabled | 0 - Disabled |
| | | 1 - Enabled |
| Test Count | The maximum number of tests per Count Period | 1 - 65,534 or a no limit value of 65,535 |

The silence pump alarm function allows the patient to silence the pump's active audible alarms. The physician may configure the parameters of this function to allow the patient to silence the alarm for a specific time period or until a new alarm occurs. Table 5 contains configuration parameters associated with the silence pump alarm function.

| **TABLE 5** | | |
|---|---|---|
| **Parameters** | **Description** | **Range** |
| Enable/ Disable | Function is available for patient's use if enabled | 0 - Disabled |
| | | 1- Enabled |
| Silence Duration | Period of time for which an active, audibly enabled alarm will remain silent | 1 - 65,534 minutes (a timed duration) or a no limit or untimed value of 65,535 |

In addition, the physician may program the pump to allow the patient to retrieve a status summary of the pump's functions. For example, the pump may be programmed to communicate status information that includes: alarms present; alarms silenced; pump stopped; reservoir low; early replacement indicator active; end of service; patient increase in progress; patient decrease in progress; physician bolus in progress; patient bolus in progress; INC/DEC lockout in progress; bolus lockout in progress; INC/DEC count at limit; bolus count at limit; telemetry successful, etc.

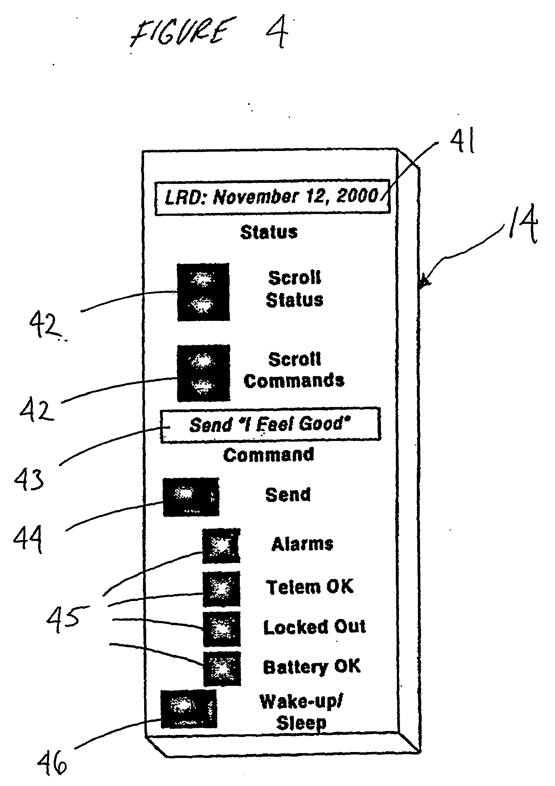

After receiving a status summary command from the patient, the pump transmits the requested information to the patient control device 14. In turn, the patient control device communicates the information to the patient. Preferably, the patient will simply receive a "yes" or "no" indication. The "yes" or "no" indication can be communicated to the patient in a number of ways, for example, LED's, LCD, text, audible tones, etc. Preferably, the patient control device has dot matrix or LCD display for displaying textual messages to the patient. Multiple indications may be present at one time and will be communicated in a single response from the pump. An exemplary embodiment of the patient control device is shown in Figure 4. Table 6 contains configuration parameters associated with the status summary function.

| **TABLE 6** | | |
|---|---|---|
| **Parameters** | **Description** | **Range** |
| Enable/Disable | Function is available for patient's use if enabled | 0 - Disabled |
| | | 1 - Enabled |
| Alarms Present | Self-Explanatory | For each: |
| Alarms Silenced | | 0 - No |
| Pump Stopped | | 1 - Yes |
| Reservoir Low | | |
| Early Replacement Indicator Active | | |
| End of Service | | |
| Patient Increase in Progress | | |
| Patient Decrease in Progress | | |
| Physician Bolus in Progress | | |
| Patient bolus in Progress | | |
| INC/DEC Lockout in Progress | | |
| Bolus Lockout in Progress | | |
| INC/DEC Count at Limit | | |
| Bolus Count at Limit | | |
| Telemetry Successful | | |

The pump mechanism of the present invention provides specific responses to each of the above-described patient commands. Specifically, after receiving a patient command the pump mechanism 10 transmits a response to the patient control device 14. In turn, the patient control device receives and communicates the pump mechanism's response to the patient. The patient control device displays the response to the patient's command in a format that is easily communicated to the patient, for example, LCD, text, audible messages, etc. As shown in Figure 4, the patient control device preferably has a dot matrix or LCD display for displaying textual messages to the patient. Exemplary pump responses include: command accepted; function disabled; function already in progress; function at rate limit; function locked out due to time; function locked out due to count, etc.

In addition, the physician or programmer can select which patient commands and resulting pump responses will be recorded as a time-stamped patient event. The defined patient commands and resulting pump responses (accept, reject) may be selectivity filtered based on the physician's configuration decisions. If the physician does not choose which events to filter, the pump mechanism's default setting will record all events. The time-stamped patient events are recorded in a patient event log contained in the pump mechanism's memory. Only the response event is recorded into the patient event log since the request is implied. In other words, there is no need to record both the request and the response. Even though the present invention is capable of time/date stamping and recording all patient events, recording more than 80 can by burdensome for the physician. Typically, 80 patient events is the number of events that a physician can analyze without being overwhelmed. In addition, the anticipated size of the event record and the related patient event log resulting from over 80 recorded patient events may comprise the amount of memory allocated to other features of the pump mechanism.

Event codes for up to 256 patient events can be supported by the pump mechanism. Preferably, event codes 0 - 127 are assigned to internally set patient events that are associated with patient commands and physician programming activity. Exemplary internally set patient events associated with patient commands include: stop pump requests; rejected stop pump requests; re-start pump requests; rejected re-start pump requests; increase current rate requests; rejected increase current rate requests; decrease current rate requests; rejected decrease current rate requests; patient bolus request; rejected patient bolus request; stop patient bolus request; rejected stop patient bolus request; status summary requests; rejected status summary requests; test pump alarm requests; rejected test pump alarm requests; silence pump alarm requests; rejected silence pump alarm requests; etc. Exemplary internally set patient events associated with physician programming activity include: changed patient configuration; clear patient configuration history; clear patient event log; etc. The other codes may be used by the programmers.

The pump mechanism 10 contains two sets of activity counters that increment each time an internally set patient event is requested by the patient. The first counter set comprise patient activity life counters that count the number of times an internally set patient event has occurred in the pump mechanism's lifetime. The pump mechanism has a patient activity life counter for each patient event described above. A patient activity life counter is incremented based on the results of the patient request associated with that patient activity life counter. Specifically, an activity life counter will increment once each time a patient's request is accepted or rejected. The patient activity life counter does not distinguish between reasons why any particular patient request is rejected (e.g., function disabled, timed lockout in progress, etc.). The counter may log up to 65,535 events - correlating to approximately 20 events per day for 9 years. The value 65,535 indicates that more that 65,535 events have occurred. Each patient activity life counter is set to zero (0) only on the initial application of the pump mechanism's battery power. Thereafter, the patient activity life counters may not be cleared by the physician or patient. The patient activity life counters may only be cleared by special manufacturing commands that are not made available to physicians or patients.

The second set of activity counters are patient activity interval counters that can be cleared by the physician and record the number of patient requests accepted or rejected by the pump since the last time that the patient activity counters were cleared. The pump mechanism has a patient activity interval counter for each patient event described above. Each time a patient activity counter is cleared by the physician the time and date of the clear is stored in the pump mechanism's memory. Similar to a patient activity life counter, a patient activity interval counter is incremented based on the results of the patient request associated with that patient activity interval counter. Specifically, an activity interval counter will increment once each time a patient's request is accepted or rejected. Like the patient activity life counter, the patient activity interval counter does not distinguish between reasons why any particular patient request is rejected (e.g., function disabled, timed lockout in progress, etc.). The counter may log up to 65,535 events - correlating to approximately 20 events per day for 9 years. Again, the value 65,535 indicates that more that 65,535 events have occurred. The patient activity life counters and the patient activity interval counters provide a valuable indication of the patient's exact activities and the amount of battery usage from commands sent to the pump and pump responses sent from the pump via telemetry.

In addition to the above described internally set patient events, event codes 128 - 255 are reserved and may be assigned to externally set patient events associated with a patient feedback mechanism that allows the patient to send information to the pump mechanism relating to the patient's personal assessment of how the patient is feeling and the patient's activity level. Specifically, the physician programs the pump to receive patient event commands that correspond to the patient's physical state or activity level (e.g., "I feel terrible right now," "I feel good right now," "I am active at this time," "I am resting at this time"). Each externally set patient event may be assigned an event code, ranging from 128 to 255, that is recognized by the pump mechanism. The pump is not required to have any knowledge of what the event code means, in other words the meaning is completely defined by the programmer and its user interface. Using the patient control device 14, the patient transmits the patient feedback to the pump mechanism. The patient control device displays textual messages or commands that the patient transmits to the pump mechanism. The information is time/date stamped according to the pump mechanism's current time/date and recorded in the patient event log contained in the pump mechanism's memory. The patient feedback information can be downloaded and reviewed by the physician at the next clinical visit. The physician can use the patient feedback mechanism to link the patient's feedback information to the infusion/therapy being delivered at the time the patient entered the information into the pump. In addition, the physician can review any infusion rate changes made by the patient and the time/date those changes were made. The patient feedback mechanism provides valuable patient information to the physician that is used at a later date without requiring the patient to remember or note the activity. After retrieving the information, the physician may clear all the time-stamped patient events in the patient event log. The physician, however, may not selectively choose which patient events to clear.

Referring to Figure 4, an exemplary patient control device 14 is depicted. The patient control device 14 may be configured with lights or LED displays 41 to communicate pump responses, pump status information, and patient feedback information to the patient. In addition, the patient control device 14 may be configured with a scroll function 42 and an LED display 43 that allows the patient to scroll through various pump patient commands or patient feedback information. For example, the patient may use the scroll function to select a desired command or feedback input. When the desired patient command or patient feedback input appears in the LED display or window (i.e., "I feel good," "Deliver Bolus," etc.), the patient can press the "send" button 44 causing the telemetry message to be sent to the pump mechanism and the status of the command or feedback input (i.e. "telemetry complete") to appear in the status window 41. The patient control device 14 may be configured with displays or light indicators 45 that provide feedback concerning system functions or the status of the pump. In addition, the patient control device 14 may include a power-saving button 46 that when pressed shuts down the displays and internal electronics until pressed again.

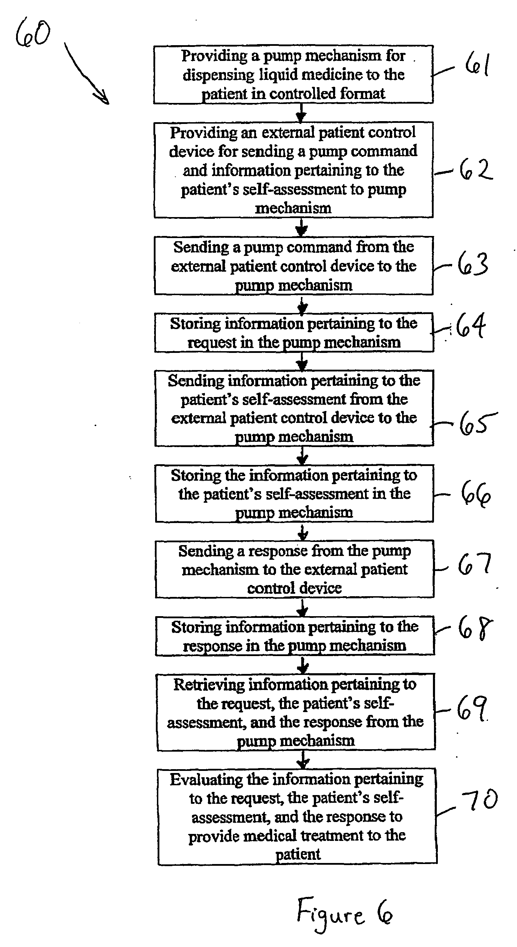

Figure 6 depicts an exemplary method 60 for linking information pertaining to a patient's physical assessment and activity level to a particular drug therapy routine. At step 61, the pump mechanism 10 is provided for dispensing medicine to the patient in a controlled format. At step 62, the external patient control device 12 is provided for sending the pump command and information pertaining to the patient's self assessment to pump mechanism 10. At step 63, a pump command (e.g., increase/decrease infusion rate) is sent from the external patient control device 12 to pump mechanism 10. At step 64, information pertaining to the request is time/date stamped and is stored in the pump mechanism 10. At step 65, information pertaining to the patient's self-assessment is sent to the pump mechanism 10 via the external patient control device 12. At step 66, the information pertaining to the patient's self-assessment is time/date stamped and stored in the pump mechanism 10. It should be understood that the order of the previous steps are not essential to the operation of the system. In other words, information pertaining to the patient's self-assessment can be sent and stored in the pump mechanism prior to sending and storing information pertaining to the patient's request. At step 67, a response from the pump mechanism 10 is sent to the external patient control device 12. The response is sent in response to the pump command. At step 68, information pertaining to the response is stored in the pump mechanism 10. At step 69, information pertaining to the pump command, the patient's self-assessment, and the response are retrieved from the control system. Finally, at step 70, the information is evaluated by a physician and used to provide medical treatment to the patient.

The pump mechanism 10 is programmed to maintain the status of all patient controlled pump functions in the memory of the control system so that the status can be readily accessible to the physician or programmer. As stated, the status information can be uploaded and analyzed by the physician. The physician can track the patient's activity and automatically adjust refill appointment dates. Table 7 contains status parameters that are maintained by the pump.

| **TABLE 7** | |
|---|---|
| Patient Alarms Silenced Status | The pump will provide an indication (Yes/No) that alarms have been silenced. |
| Patient Alarms Silence Time Remaining Status | The pump will provide a value indicating the time remaining in the Silence Duration (Range: 0-65,534 minutes, where 65,535 indicates Silent Until Manually Re-enabled). |
| Patient Count Period Remaining Status | The pump will provide a value indicating the time remaining in the count period (Range: 0-65,534 minutes or a 65,535 value indicating that the count period is not timed, or "no limit"). |
| Patient Stop in Progress Status | The pump will provide an indication (Yes/No) whether the pump has been stopped using a patient stop command. |
| Patient Stop Lockout Time Remaining Status | The pump will provide a value indicating the time remaining in the stop lockout (Range: 0-65,534 minutes or a 65,535 value indicating "No Limit"). |
| Patient Stop Duration Remaining Status | The pump will provide a value which indicates the time remaining in the Stop Duration (Range: 0-65,534 minutes, where 65,535 indicates "No Limit"). |
| Patient Stop Count within Count Period Status | The pump will provide a value which indicates the number of stop commands used within the current count period (Range: 0-65,534, where 65,535 indicates "65,535 or greater"). |
| Patient Stop Locked out due to Count within Period Status | The pump will provide an indication (Yes/No) whether the patient's Stop command is currently locked out due to the number of stop commands used within the count period. |
| Patient INC/DEC Rate Index | The pump will provide a value which |
| Status | indicates the index of the current INC/DEC Rate in the patient configuration (Range: 0-10, where a separate value indicates that INC/DEC rates are not being used are disabled). |
| Patient INC/DEC Lockout Time Remaining Status | The pump will provide a value which indicates the time remaining in the INC/DEC Lockout (Range: 0-65,534 minutes, where 65,535 indicates "No Limit"). |
| Patient INC/DEC Rate Deviation within Count Period Status | The pump will provide a value which indicates the number rates the patient has deviated from the reference within the current Count Period Range: -10 to +10, where a separate value indicates that INC/DEC Rates are not being used: INC/DEC is disabled. |
| Patient INC Locked out due to Max Rate Deviation within Count Period Status | The pump will provide an indication (Yes/No) whether the patient's INC command is currently locked out due to the number of rate deviations within the count period. |
| Patient DEC Locked out due to Max Rate Deviation within Count Period Status | The pump will provide an indication (Yes/No) whether the patient's DEC command is currently locked out due to the number of rate deviations within the count period. |
| Patient Bolus in Progress Status | The pump will provide an indication (Yes/No) whether a bolus is in progress due to a patient bolus command. |
| Patient Current Bolus Rate Status | The pump will provide a value which indicates the current bolus rate (Range: any of the valid rates supported by the pump "Not Applicable" if a bolus is not in progress). |
| Patient Bolus Lockout Time Remaining Status | The pump will provide a value which indicates the time remaining in the bolus duration (Range: 0-65,534 minutes, where 65,535 indicates "No Limit"). |
| Patient Bolus Duration Remaining Status | The pump will provide a value which indicates the time remaining in the Bolus Duration (Range: 0-65,534 minutes, where 65,535 indicates "No Limit"). |
| Patient Bolus Count within Count Period Status | The pump will provide a value which indicates the number of bolus commands |
| | used, within the current count period (Range; 0-65,534, where 65,535 indicates "65,535 or greater"). |
| Patient Bolus Locked Out due to Count within Count Period Status | The pump will provide an indication (Yes/No) whether the patient's bolus command is currently locked out due to the number of bolus commands used within the count period. |
| Patient Test Pump Alarm Locked Out due to Count within Count Period Status | The pump will provide an indication (Yes/No) whether the patient's test alarm command is currently locked out due to the number of test alarm commands used within the count period. |

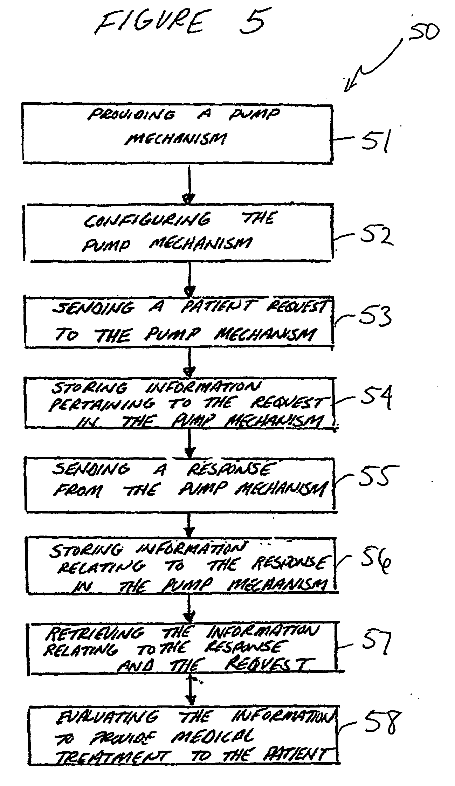

Figure 5 depicts a therapeutic method 50 of administering liquid medicine to a patient by infusion using the drug delivery system of the present invention. At step 51, a pump mechanism for dispensing medicine to a patient in a format controlled by a control system within the pump mechanism is provided. At step 52, an external programming device is used to configure the control system of the pump mechanism. At step 53, an external patient control device is used to send a patient request to the control system and to receive a response from the control system. At step 54, the information pertaining to the request is stored in the control system for a predetermined period of time. At step 55, a response from the control system is sent to the external patient control device. At step 56, information pertaining to the response is stored in the control system for a predetermined period of time. At step 57, the information pertaining to the request and the response is retrieved from the control system. At step 58, the information pertaining to the request and the response is evaluated to provide medical treatment to the patient.

## Claims

1. A drug delivery system for delivering medicine comprising, in combination:
a pump mechanism (10) having a control system (20) for controlling the pumping rate of the pump mechanism (10), the control system (20) programmed to have a plurality of pumping rates including a reference pumping rate and to allow a patient to select a desired pumping rate from the plurality of pumping rates, the control system (20) programmed to prevent the patient from deviating from the reference pumping rate by a prescribed amount within a prescribed period of time;
an external programming device (12) for programming the control system (20); and
an external patient control device (14) for sending patient commands to the control system (20) and for receiving responses from the control system (20), the external patient control device (14) including a display console for communicating the response from the control system (20) to the patient; and
**characterized by** further having a patient feedback mechanism in the control system (20) for receiving and storing personal assessment information sent from the patient via the external patient control device (14).

2. The drug delivery system of claim 1 further comprising a patient event log in the control system (20) for storing information pertaining to the patient commands to the control system (20) and the responses from the control system (20).

3. The drug delivery system of any of claims 1-2 further comprising a patient activity counter mechanism for counting the number of patient requests sent to the control system (20) and the number of responses sent from the control system (20).

4. The drug delivery system of any of claims 1-3 wherein the control system (20) is further programmed to accept a prescribed number of patient bolus injection commands within a prescribed period of time.

5. The drug delivery system of any of claims 1-4 wherein the control system (20) is further programmed to allow the patient to change between the pumping rates a prescribed number of times within a prescribed period of time.

6. The drug delivery system of any of the claims 1-5 wherein the control system (20) is programmed to communicate status information for the pump mechanism (10) to the external patient control device (14), the status information selected from the group consisting of alarms present, alarms silenced, pump stopped, reservoir low, patient increase in progress, patient decrease in progress, physician bolus in progress, patient bolus in progress, INC/DEC lockout in progress, bolus lockout in progress, INC/DEC count at limit, bolus count at limit, and telemetry successful.

7. The drug delivery system of any of claims 1-6 wherein the patient commands are selected from the group consisting of start pump, stop pump, increase pump rate, decrease pump rate, start bolus injection, stop bolus injection, test pump alarm, silence pump alarm, un-silence pump alarm, and provide pump status.

8. The drug delivery system of any of claims 1-7 wherein the responses from the control system (20) are selected from the group consisting of command accepted, function disabled, function already in progress, function at rate limit, function locked out due to time, and function locked out due to count.

## Patentansprüche

1. Arzneimittelabgabesystem zum Abgeben einer Medizin, das in Kombination folgendes umfassst:
einen Pumpmechanismus (10) mit einem Steuersystem (20) zum Steuern der Pumprate des Pumpmechanismus (10), wobei das Steuersystem (20) so programmiert ist, dass es eine Mehrzahl von Pumpraten einschließlich einer Referenzpumprate aufweist und dass es einem Patienten ermöglicht, eine gewünschte Pumprate aus der Mehrzahl von Pumpraten auszuwählen, und wobei das Steuersystem (20) programmiert ist, um zu verhindern, dass der Patient innerhalb eines vorgeschriebenen Zeitraums um einen vorgeschriebenen Betrag von der Referenzpumprate abweicht,
eine externe Programmiervorrichtung (12) zum Programmieren des Steuersystems (20) und
eine externe Patientensteuervorrichtung (14) zum Senden von Patientenbefehlen zu dem Steuersystem (20) und zum Empfangen von Antworten von dem Steuersystem (20), wobei die externe Patientensteuervorrichtung (14) eine Anzeigekonsole zum Übermitteln der Antwort von dem Steuersystem (20) zu dem Patienten aufweist,
**dadurch gekennzeichnet, dass** es des weiteren einen Patientenrückmeldungsmechanismus in dem Steuersystem (20) aufweist, um von dem Patienten über die externe Patientensteuervorrichtung (14) gesendete persönliche Beurteilungsinformationen zu empfangen und zu speichern.

2. Arzneimittelabgabesystem nach Anspruch 1, das des weiteren ein Patientenereignisprotokoll in dem Steuersystem (20) aufweist, um Informationen, die zu den Patientenbefehlen für das Steuersystem (20) und zu den Antworten von dem Steuersystem (20) gehören, zu speichern.

3. Arzneimittelabgabesystem nach einem der Ansprüche 1 bis 2, das des weiteren einen Patientenaktivitätszählermechanismus zum Zählen der Anzahl der zu dem Steuersystem (20) gesendeten Patientenanfragen und der Anzahl der von dem Steuersystem (20) gesendeten Antworten aufweist.

4. Arzneimittelabgabesystem nach einem der Ansprüche 1 bis 3, wobei das Steuersystem (20) des weiteren programmiert ist, um eine vorgeschriebene Anzahl von Patientenbolusinjektionsbefehlen innerhalb eines vorgeschriebenen Zeitraums zu empfangen.

5. Arzneimittelabgabesystem nach einem der Ansprüche 1 bis 4, wobei das Steuersystem (20) des weiteren programmiert ist, um dem Patienten zu ermöglichen, innerhalb eines vorgeschriebenen Zeitraums eine vorgeschriebene Anzahl von Malen die Pumpraten zu wechseln.

6. Arzneimittelabgabesystem nach einem der Ansprüche 1 bis 5, wobei das Steuersystem (20) programmiert ist, um Statusinformationen für den Pumpmechanismus (10) zu der externen Patientensteuervorrichtung (14) zu übermitteln, wobei die Statusinformationen aus der Gruppe bestehend aus Alarme vorhanden, Alarme unterdrückt, Pumpe angehalten, Reservoir niedrig, Patientenerhöhung findet statt, Patientenverringerung findet statt, Arztbolus findet statt, Patientenbolus findet statt, INC/DEC-Ausschluss findet statt, Bolusausschluss findet statt, INC/DEC-Zählwert an der Grenze, Boluszählwert an der Grenze und Telemetrie erfolgreich ausgewählt werden.

7. Arzneimittelabgabesystem nach einem der Ansprüche 1 bis 6, wobei die Patientenbefehle aus der Gruppe bestehend aus Pumpe starten, Pumpe anhalten, Pumprate erhöhen, Pumprate verringern, Bolusinjektion starten, Bolusinjektion unterbrechen, Pumpenalarm testen, Pumpenalarm unterdrücken, Unterdrückung des Pumpenalarms aufheben und Pumpenstatus bereitstellen ausgewählt werden.

8. Arzneimittelabgabesystem nach einem der Ansprüche 1 bis 7, wobei die Antworten von dem Steuersystem (20) aus der Gruppe bestehend aus Befehl angenommen, Funktion deaktiviert, Funktion wird bereits ausgeführt, Funktion an der Ratengrenze, Funktion aus Zeitgründen ausgeschlossen und Funktion aus Zählwertgründen ausgeschlossen ausgewählt werden.

## Revendications

1. Système d'administration de médicaments pour administrer un médicament comprenant, en combinaison :
un mécanisme à pompe (10) ayant un système de commande (20) pour commander le débit de pompage du mécanisme à pompe (10), le système de commande (20) étant programmé pour présenter une pluralité de débits de pompage qui incluent un débit de pompage de référence, et pour permettre à un patient de choisir un débit de pompage désiré à partir de la pluralité de débits de pompage, le système de commande (20) étant programmé pour empêcher au patient de s'écarter du débit de pompage de référence à raison d'une quantité prescrite dans une période temporelle prescrite ;
un dispositif de programmation externe (12) pour programmer le système de commande (20) ; et
un dispositif de commande patient externe (14) pour envoyer des ordres du patient au système de commande (20) et pour recevoir les réponses provenant du système de commande (20); le dispositif de commande patient externe (14) incluant une console d'affichage pour communiquer la réponse provenant du système de commande (20) vers le patient ; et
**caractérisé en ce qu'**il comprend en outre un mécanisme de rétroaction patient dans le système de commande (20) pour recevoir et stocker des informations de jugement personnel envoyées depuis le patient via le dispositif de commande de patient externe (14).

2. Système d'administration de médicaments selon la revendication 1, comprenant en outre un procès-verbal d'événements patient dans le système de commande (20) pour mémoriser des informations appartenant aux ordres du patient vers le système de commande (20) et les réponses provenant du système de commande (20).

3. Système d'administration de médicaments selon l'une des revendications 1 et 2, comprenant en outre un mécanisme de comptage d'activité patient pour compter le nombre de requêtes du patient envoyées au système de commande (20) et le nombre de réponses envoyées depuis le système de commande (20).

4. Système d'administration de médicaments selon l'une quelconque des revendications 1 à 3, dans lequel le système de commande (20) est en outre programmé pour accepter un nombre prescrit d'ordres d'injection de doses unitaires vers le patient dans une période temporelle prescrite.

5. Système d'administration de médicaments selon l'une quelconque des revendications 1 à 4, dans lequel le système de commande (20) est en outre programmé pour permettre au patient de changer entre les débits de pompage un nombre de fois prescrits dans une période temporelle prescrite.

6. Système d'administration de médicaments selon l'une quelconque des revendications 1 à 5, dans lequel le système de commande (20) est programmé pour communiquer des informations d'état pour le mécanisme de pompe (10) vers le dispositif de commande patient externe (14), les informations d'état étant choisies parmi le groupe comprenant les alarmes présentes, les alarmes silencieuses, l'arrêt de la pompe, un niveau bas dans le réservoir, l'augmentation des progrès du patient, la diminution des progrès du patient, une dose unitaire administrée par le médecin en cours, une dose unitaire choisie par le patient en cours, des interruptions INC/DEC en cours, des interruptions de dose unitaire en cours, l'arrivée du décompte INC/DEC à la limite, l'arrivée du décompte de dose unitaire à la limite, et le succès des opérations de télémétrie.

7. Système d'administration de médicaments selon l'une quelconque des revendications 1 à 6, dans lequel les ordres du patient sont choisis parmi le groupe comprenant le démarrage de la pompe, l'arrêt de la pompe, l'augmentation du débit de pompe, la diminution du débit de pompe, le départ de l'injection d'une dose unitaire, l'arrêt de l'injection d'une dose unitaire, le test de l'alarme de la pompe, la mise au silence de l'alarme de la pompe, l'annulation de cette mise au silence, et la fourniture de l'état de pompe.

8. Système d'administration de médicaments selon l'une quelconque des revendications 1 à 7, dans lequel les réponses provenant du système de commande (20) sont choisies parmi le groupe comprenant l'acceptation d'un ordre, l'annulation d'une fonction, l'existence d'une fonction en cours, l'arrivée d'une fonction à un taux limite, le verrouillage d'une fonction en raison du temps, et le verrouillage d'une fonction en raison du décompte.
